# EUROPEAN PATENT SPECIFICATION

(11) **EP 3 025 686 B1**
(45) Date of publication and mention of the grant of the patent: **28.02.2018**
(21) Application number: 15173337.5
(22) Date of filing: 23.06.2015
(51) Int. Cl.: A61F 2/966

(54) **STENT DELIVERY SYSTEM**
STENTTRÄGERSYSTEM
SYSTEME D'ADMINISTRATION DE STENT

(30) Priority: 29.11.2014 JP 2014242633
(43) Date of publication of application: 01.06.2016
(73) Proprietor: PENTAS Inc., Tokyo, 150-0002 (JP)
(72) Inventor: NISHIGISHI, Makoto c/o ASAHI INTECC CO., LTD., Nagoya-shi, Aichi 463-0024 (JP)
(74) Representative: TBK

(56) References cited:
- EP-A1- 1 374 801
- EP-A1- 2 543 345
- US-A1- 2009 270 974

## Description

### TECHNICAL FIELD

The present invention relates to a stent delivery system for delivering a stent stored in a catheter to a target site with a pusher guide wire.

### BACKGROUND ART

Stents are medical devices used for treating a stenosis or occlusion and aneurysm (hereinafter referred to as a "target site") formed in a blood vessel or a digestive organ. For example, a stent may be used for supporting the lumen of a blood vessel or a digestive organ so that the blood vessel or the digestive organ expanded with a balloon catheter will not be affected with stenosis again. Further, a stent may be used for confining an embolization coil in an aneurysm formed in an arterial blood vessel of the abdomen or the brain to prevent the aneurysm from rupturing.

Stents are generally classified into two categories: a balloon-expandable stent which is expandable with a balloon catheter and a self-expandable stent in which the stent itself is expandable spontaneously. Recently, self-expandable stents resistant to deformation even when subjected to external force have been often used.

As a stent delivery system for delivering a stent stored inside a catheter to a target site, known are a system in which an anchor member provided in a stent is disposed within a gap formed between a middle columnar member and a proximal end columnar member in order to push the stent in the direction of the front end (for example, see Patent Literature 1 below) and a system in which a metal ring is separately provided at the outer periphery of a coil body of a pusher guide wire in order to push a stent in the direction of the front end (for example, see Patent Literature 2 below).

However, there has been the following problem with the stent delivery system according to Patent Literature 1. Although the proximal end columnar member can make contact with the anchor member to deliver the stent in the direction of the front end when an operator pushes the pusher guide wire in the direction of the front end, the rotating force of the pusher guide wire is difficult to be transmitted to the front end of the pusher guide wire when the operator rotates the pusher guide wire, because the middle columnar member is apart from the proximal end columnar member. As a result, it is difficult to precisely release the stent at the target site (in other words, the stent is anteroposteriorly dislocated from the target site).

Further, there has been the following problem with the stent delivery system according to Patent Literature 2. Although the rotating force exerted by an operator can be transmitted to the front end of the pusher guide wire since the coil body of the pusher guide wire is not separated but integrally formed until the front end, the flexibility of the pusher guide wire is impaired since the metal ring for pushing the stent in the direction of the front end is separately provided. In particular, there has been a problem that the metal ring attached separately is caught up within the catheter when inserting the stent delivery system into a curved blood vessel or digestive organ, resulting in a difficult delivery of the stent to the target site.

### CITATION LIST

### Patent Literature

Patent Literature 1: Japanese Patent No. 4574131
Patent Literature 2: Japanese Patent Laid-Open No. 2013-521022

### SUMMARY OF THE INVENTION

### TECHNICAL PROBLEM

The present invention is made in view of these circumstances. An object of the present invention is to provide a stent delivery system in which the flexibility of a pusher guide wire is not impaired, and a rotating force exerted by an operator can be transmitted to the front end of the pusher guide wire, and thus the stent can be easily and precisely released at a target site.

### SOLUTION TO PROBLEM

The above problems can be solved by the means listed below.

An aspect 1 of the present invention is a stent delivery system for delivering a stent having an anchor member at a proximal end part to a target site with a pusher guide wire, the pusher guide wire comprises a core shaft; a coil body that covers a front end part of the core shaft and that includes a tapered portion having an outer diameter decreasing toward a front end; and a protruded portion that fixes to an outer periphery of the coil body at a front end side relative to the tapered portion and that is capable of contacting on the anchor member, wherein the anchor member is positioned between the tapered portion and the protruded portion when the stent is delivered with the pusher guide wire.

The stent delivery system may further comprise a catheter into which the pusher guide wire holding the stent can be inserted.

An aspect 2 of the present invention is the stent delivery system according to aspect 1, wherein the anchor member is inclined in the same direction as the tapered portion of the coil body relative to an axis of the core shaft.

An aspect 3 of the present invention is the stent delivery system according to aspect 1 or 2, wherein a resin layer is provided on an outer periphery of the tapered portion.

### ADVANTAGEOUS EFFECT OF THE INVENTION

In the stent delivery system according to aspect 1 of the present invention, the pusher guide wire comprises a coil body having a tapered portion in which the outer diameter decreases toward a front end, and the protruded portion is fixed to the outer periphery of the above coil body. The anchor member of the stent is arranged between the tapered portion of the coil body and the protruded portion when the stent is delivered to the target site with the pusher guide wire. The flexibility of the pusher guide wire is not impaired since the anchor member can be pushed in the direction of the front end with the tapered portion of the coil body without separately providing a metal ring. Further, a rotating force exerted by an operator can be transmitted to the front end of the pusher guide wire since the coil body extends to the front end of the pusher guide wire. Moreover, the stent can be retracted into the catheter in the middle of release by pulling the pusher guide wire in the direction toward the proximal end and allowing the protruded portion to make contact with the anchor member. This can reduce a risk that the pusher guide wire is caught up inside the catheter when the stent delivery system is inserted into a curved blood vessel or digestive organ, and the stent can be more easily released at a position at which an operator aims.

In the stent delivery system according to aspect 2 of the present invention, the anchor member is inclined in the same direction as the tapered portion of the coil body relative to the axis of the core shaft. Therefore, the tapered portion of the coil body can make contact with the outer periphery of the anchor member when an operator pushes the pusher guide wire in the direction of the front end to increase the contact area between the tapered portion of the coil body and the anchor member. As a result, even when the stent delivery system is inserted into a curved blood vessel or digestive organ, the stent can be easily delivered to the target site with the pusher guide wire.

In the stent delivery system according to aspect 3 of the present invention, a resin layer is provided on the outer periphery of the tapered portion. Therefore, this can reduce a risk that the anchor member is caught up between wires of the coil body when an operator pushes the pusher guide wire in the direction of the front end. As a result, the stent can be more reliably delivered to the target site.

### BRIEF DESCRIPTION OF THE DRAWINGS

Fig. 1 shows an overall view of a stent delivery system according to an embodiment of the present invention. Fig. 1A shows a state in which the stent is stored inside the catheter. Fig. 1B shows a state in which the pusher guide wire is pushed in the direction of the front end to release the stent from the front end of the catheter. Fig. 1C shows a state in which the pusher guide wire is pulled back in the direction toward the proximal end to retract the stent into the catheter in the middle of release.
Fig. 2 shows an enlarged view of Portion A in Fig. 1.
Fig. 3 shows a first variation of Fig. 2, illustrating a part of a stent delivery system according to a second embodiment.
Fig. 4 shows a second variation of Fig. 2, illustrating a part of a stent delivery system according to a third embodiment.
Fig. 5 shows a first variation of Fig. 1A, illustrating an overview of a stent delivery system according to a fourth embodiment.

### DESCRIPTION OF EMBODIMENTS

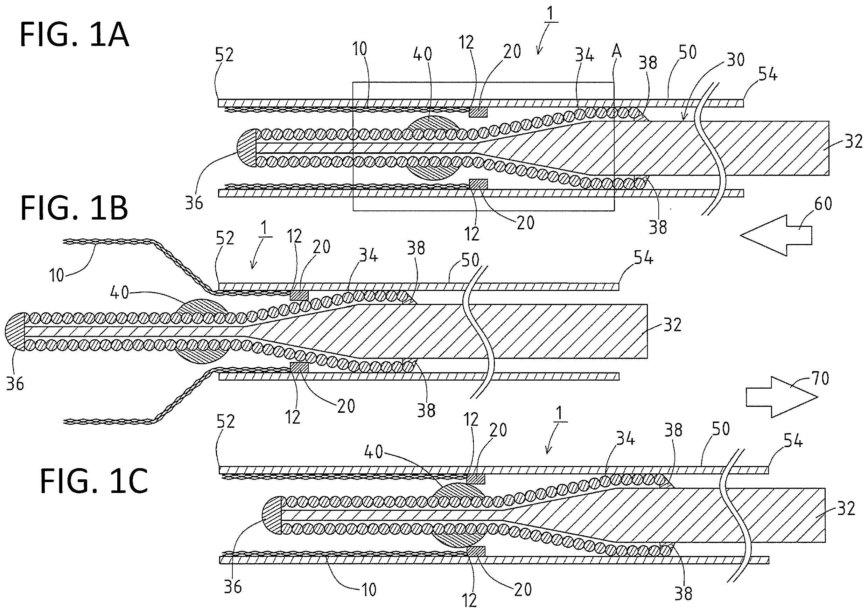

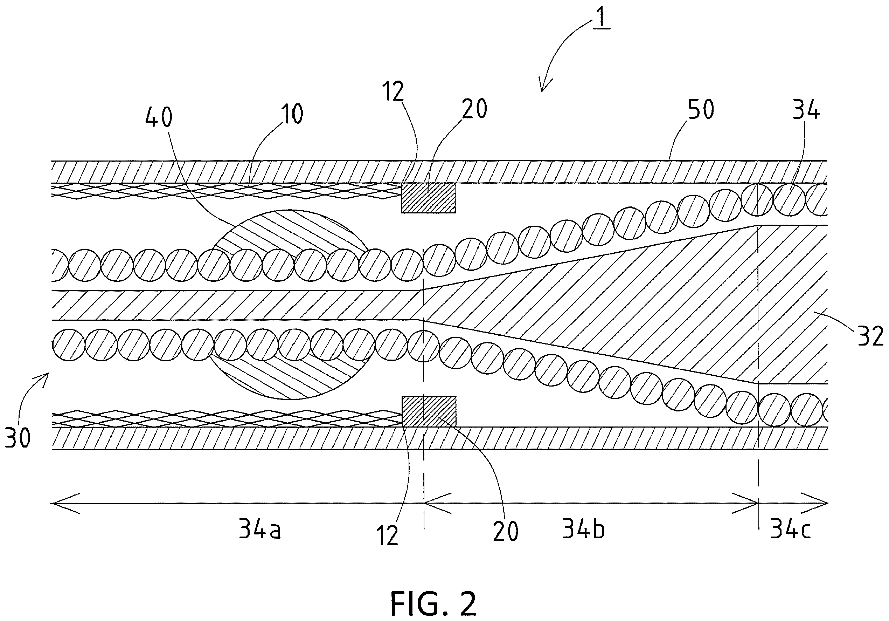

First, a stent delivery system 1 according to an embodiment of the present invention is described with reference to Figs. 1A to 1C and Fig. 2. In Figs. 1A to 1C and Fig. 2, the left side corresponds to the front side (the distal side) which is to be inserted into the body, and the right side corresponds to the proximal side (the base end side) which is to be operated by an operator such as a physician. Note that Fig. 2 shows an enlarged view of Portion A in Fig. 1A.

As shown in Fig. 1A, a stent delivery system 1 for delivering a stent 10 to the target site with a pusher guide wire 30 comprises the stent 10, the pusher guide wire 30 and a catheter 50.

Publicly known stents can be used for the stent 10. For example, any of the following can be used: those in which multiple wires are interwoven into a web-like structure (a mesh-like structure) and those in which multiple struts (wavy annular bodies) are axially arranged, and adjacent struts (wavy annular bodies) are connected via a connecting region. Moreover, a bare metal stent with no drug applied on a surface thereof as well as a drug-elution stent (DES) with a drug applied on a surface thereof can be used as the stent 10.

According to an embodiment of the present invention, the stent 10 is formed with metal wires. For example, stainless steel, W, Pt, a Pt-Ni alloy, a Co-Cr alloy, a Ni-Ti alloy, a Cu-Al-Ni alloy and the like can be used for the metal wires. However, the stent 10 may be formed with a resin material such as polyester, polyurethane, polyolefine, polytetrafluoroethylene, a silicon resin and the like instead of only metal wires.

An anchor member 20 is attached to a proximal end part 12 of the stent 10. The anchor member 20 is provided in order for the anchor member 20 to prevent the stent 10 from moving anteroposteriorly from the target site or rotating in a radial direction upon making contact with a blood vessel wall or a digestive organ wall when the stent 10 is expanded at the target site. The anchor member 20 comprises a hollow cylinder body such as a ring or a coil body.

The pusher guide wire 30 comprises a core shaft 32, a coil body 34 covering a front end part of the core shaft 32, a front end joining portion 36 joining the front end of the core shaft 32 with the front end of the coil body 34, a proximal end joining portion 38 joining the proximal end of the coil body 34 with the core shaft 32 and a protruded portion 40 fixed to the outer periphery of the coil body 34 and that is capable of making contact with the anchor member 20 of the stent 10.

As shown in Fig. 2, the coil body 34 comprises a small diameter portion 34a having a small outer diameter, a tapered portion 34b having an outer diameter decreasing toward the front end (toward the left in the figure) and a large diameter portion 34c having a large outer diameter. The small diameter portion 34a and the large diameter portion 34c are coil bodies each having a substantially constant outer diameter. The protruded portion 40 is positioned in the front end side relative to the tapered portion 34b of the coil body 34, and is fixed to the outer periphery of the small diameter portion 34a of the coil body 34. Therefore, the anchor member 20 of the stent 10 is positioned between the tapered portion 34b of the coil body 34 and the protruded portion 40.

Again, as shown in Fig. 1A, the catheter 50 is a hollow cylinder body into which the pusher guide wire 30 can be inserted. The stent 10 can be released from a front end opening 52 of the catheter 50 to the target site by inserting the pusher guide wire 30 holding the stent 10 through a proximal end opening 54 of the catheter 50, and pushing the core shaft 32 in the direction of the front end (in the direction of an arrow 60). Note that any known catheter can be used as the catheter 50.

Next, the stent delivery system 1 for delivering the stent 10 stored in the catheter 50 to the target site with the pusher guide wire 30 is described with reference to Figs. 1A to 1C.

An operator inserts the catheter 50 so that the front end opening 52 of the catheter 50 coincides with the target site. The pusher guide wire 30 is inserted through the proximal end opening 54 of the catheter 50 while catheter 50 is held not to move, and the core shaft 32 is pushed in the direction of the front end (in the direction of the arrow 60) to store the stent 10 near the front end opening 52 of the catheter 50 (see Fig. 1A). After confirming once again whether the front end opening 52 of the catheter 50 coincides with the target site, the core shaft 32 is further pushed in the direction of the front end (in the direction of the arrow 60) to release the stent 10 to the target site through the front end opening 52 of the catheter 50 (see Fig. 1B). At this time, the stent 10 can be pushed in the direction of the front end (in the direction of the arrow 60) by allowing the tapered portion 34b of the coil body 34 to make contact with the anchor member 20 of the stent 10.

However, in a case where the target site is located in a curved blood vessel or digestive organ, and thus the front end part of the catheter 50 is bent, the front end opening 52 of the catheter 50 may move in the direction of the front end (in the direction of the arrow 60) when the core shaft 32 is strongly pushed in the direction of the front end (in the direction of the arrow 60), and the release position of the stent 10 may deviate from the target site. Further, an operator may notice that the release position of the stent 10 deviates from the target site during the release of the stent 10 from the front end opening 52 of the catheter 50. Moreover, the stent 10 expands in the radial direction and at the same time becomes short in the axial direction when released from the front end opening 52 of the catheter 50. Therefore, the release position of the stent 10 may deviate from the target site when the lumen of a blood vessel or digestive organ is larger than expected. In such a case, it is necessary to pull the stent 10 in the middle of releasing back into the catheter 50 to perform the procedure again.

Upon noticing that the release position of the stent 10 is off from the target site, an operator can retract the stent 10 into the catheter 50 in the middle of release and perform the procedure again by pulling the core shaft 32 in the direction of the proximal end (in the direction of an arrow 70) and allowing the protruded portion 40 to make contact with the anchor member 20 of the stent 10 (see Fig. 1C).

As described above, according to the stent delivery system 1, the pusher guide wire 30 comprises the coil body 34 having the tapered portion 34b with an outer diameter decreasing toward the front end, and the protruded portion 40 is fixed to the outer periphery of the coil body 34. When the stent 10 is delivered to the target site with the pusher guide wire 30, the anchor member 20 of the stent 10 is positioned between the tapered portion 34b of the coil body 34 and the protruded portion 40. Therefore, the anchor member 20 can be pushed in the direction of the front end (in the direction of the arrow 60) with the tapered portion 34b of the coil body 34 without separately providing a metal ring for pushing the stent 10 in the direction of the front end as in the conventional way. As a result, the flexibility of the pusher guide wire 30 can be maintained.

Further, since the coil body 34 extends to the front end joining portion 36 of the pusher guide wire 30, a rotating force exerted by an operator can be transmitted to the front end of the pusher guide wire 30. Moreover, the stent 10 in the middle of release can be retracted into the catheter 50 by pulling back the pusher guide wire 30 in the direction of the proximal end (in the direction of the arrow 70) and allowing the protruded portion 40 to make contact with the anchor member 20. This can reduce a risk that the pusher guide wire 30 is caught up within the catheter 50 even in a case where the stent delivery system 1 is inserted in a curved blood vessel or digestive organ, allowing easy release of the stent 10 to a position at which an operator aims.

Next, materials for each member constituting the pusher guide wire 30 according to the embodiment of the present invention are described. However, there is no particular limitation for the materials.

The core shaft 32 may be formed with a stainless steel (sus304, sus316 and the like) and a superelastic alloy such as a Ni-Ti alloy.

The coil body 34 may be formed with an wire having radiopacity. Examples include gold, platinum, tungsten and alloys comprising these elements. In a case where the coil body 34 is formed with a radiopaque wire, an operator can detect the position of the coil body 34 under radiography imaging. As a result, the position of the stent 10 stored in the catheter 50 can be estimated.

Note that the coil body 34 may be formed with an wire comprising a single wire, or may be formed with a twisted wire in which multiple wires are twisted. The coil body 34 is preferably formed with a twisted wire since it is superior to a single wire in properties such as flexibility and restorability.

The front end joining portion 36 and the proximal end joining portion 38 may be formed with a solder material (such as aluminium-alloy solder, silver solder and gold solder), a metal solder (such as an Au-Sn alloy) and the like.

The protruded portion 40 may be formed with a solder material (such as aluminium-alloy solder, silver solder and gold solder), a metal solder (such as an Au-Sn alloy) and the like as in the front end joining portion 36 and the proximal end joining portion 38. Further, the protruded portion 40 may be formed by covering the outer periphery of the small diameter portion 34a of the coil body 34 with a hollow cylinder body such as a ring and a coil body.

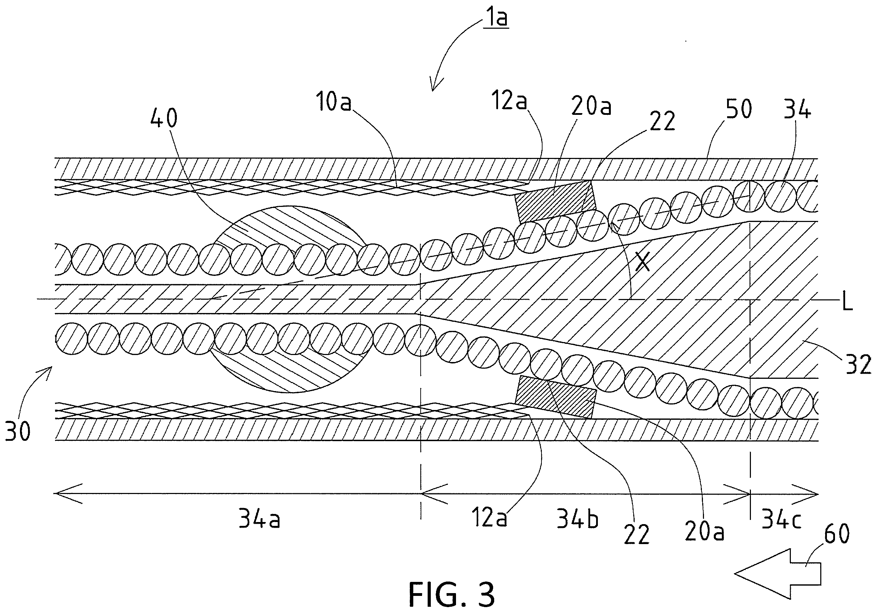

Next, the stent delivery system 1a according to the second embodiment will be described with reference to Fig. 3. Note that as in Fig. 2, the left side in Fig. 3 corresponds to the front side (the distal side) which is to be inserted into the body, and the right side corresponds to the proximal side (the base end side) which is to be operated by an operator such as a physician.

Only differences from the stent delivery system 1 shown in Fig. 2 will be described. According to the stent delivery system 1a, an anchor member 20a attached to a proximal end part 12a of a stent 10a is inclined in the same direction X as a tapered portion 34b of the coil body 34 relative to the axis L of the core shaft 32. Therefore, when an operator pushes the pusher guide wire 32 in the direction of the front end (in the direction of the arrow 60), the tapered portion 34b of the coil body 34 can make contact with an outer periphery surface 22 of the anchor member 20a to increase a contact area between the tapered portion 34b of the coil body 34 and the anchor member 20a. As a result, the stent 10a can be more easily delivered to the target site with the pusher guide wire 30 even in a case where the stent delivery system 1a is inserted into a curved blood vessel or digestive organ.

Note that uneven shapes may be formed on an outer periphery surface 22 of the anchor member 20a in order to increase a contact area with the tapered portion 34b of the coil body 34, although this is not shown in Fig. 3.

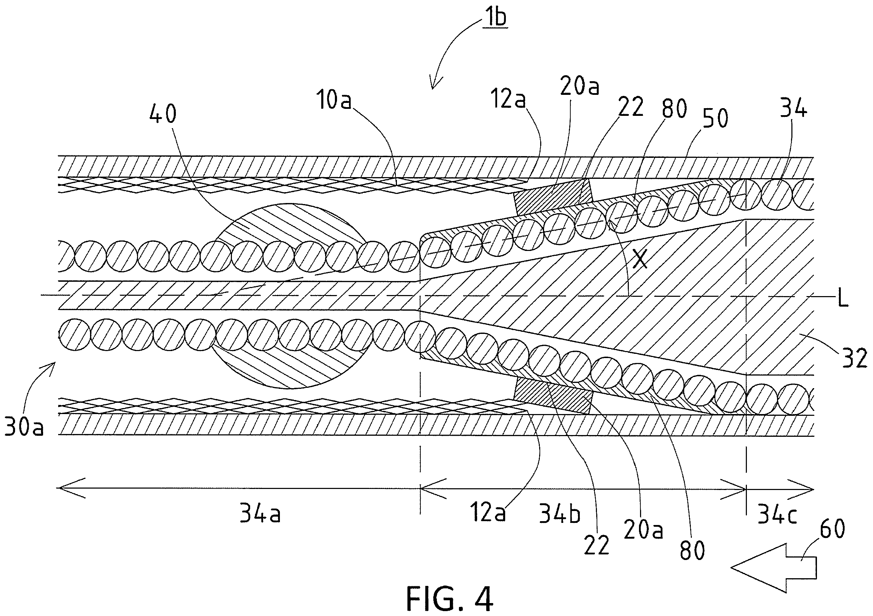

Next, a stent delivery system 1b according to a third embodiment of the present invention will be described with reference to Fig. 4. Note that as in Fig. 3, the left side in Fig. 4 corresponds to the front side (the distal side) which is to be inserted into the body, and the right side corresponds to the proximal side (the base end side) which is to be operated by an operator such as a physician.

Only differences from the stent delivery system 1a shown in Fig. 3 will be described. According to the stent delivery system 1b, a pusher guide wire 30a comprises a resin layer 80 on the outer periphery of a tapered portion 34b of the coil body 34. Therefore, a risk can be reduced that the anchor member 20a is caught up between the wires in the tapered portion 34b of the coil body 34 when an operator pushes the pusher guide wire 30a in the direction of the front end (in the direction of the arrow 60). As a result, the stent 10 can be more reliably delivered to the target site. Further, the stent 10a can be even more easily delivered to the target site with the pusher guide wire 30a even in a case where the stent delivery system 1b is inserted into a curved blood vessel or digestive organ since the contact area between the outer periphery surface 22 of the anchor member 20a and the resin layer 80 can be further increased.

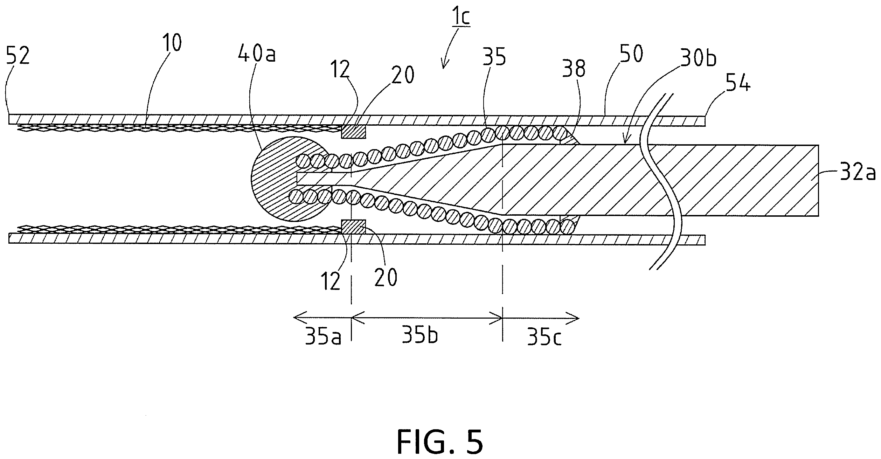

Finally, a stent delivery system 1c according to a fourth embodiment of the present invention will be described with reference to Fig. 5. Note that as in Fig. 1A, the left side in Fig. 5 corresponds to the front side (the distal side) which is to be inserted into the body, and the right side corresponds to the proximal side (the proximal side, the base end side) which is to be operated by an operator such as a physician.

Only differences from the stent delivery system 1 shown in Fig. 1A will be described. According to the stent delivery system 1c shown in Fig. 5, a pusher guide wire 30b comprises a core shaft 32a, a coil body 35 covering a front end part of the core shaft 32a, a protruded portion 40a joining the front end of the core shaft 32a with the front end of the coil body 35 and a proximal end joining portion 38 joining the proximal end of the coil body 35 with the core shaft 32a. The coil body 35 comprises a small diameter portion 35a having a small outer diameter, a tapered portion 35b having an outer diameter decreasing toward the front end (the left side in the figure) and a large diameter portion 35c having a large outer diameter.

Unlike the pusher guide wire 30, in the case of the pusher guide wire 30b, the front end joining portion 36 and the protruded portion 40 of the pusher guide wire 30 are integrally formed to provide a protruded portion 40a. Therefore, according to the stent delivery system 1c, the production of the pusher guide wire 30b is easier because the protruded portion 40 for pulling back the stent 10 in the middle of release into the catheter 50 needs not to be fixed to the outer periphery of the small diameter portion 34a of the coil body 34 as in the pusher guide wire 30.

Note that as in the pusher guide wire 30b, the protruded portion 40a in which the front end joining portion 36 and the protruded portion 40 of the pusher guide wires 30, 30a are integrally formed may also be used for the pusher guide wire 30 of the stent delivery system 1a shown in Fig. 3 and the pusher guide wire 30a of the stent delivery system 1b shown in Fig. 4.

Further, although the anchor member 20, 20a is attached only to the proximal end part 12, 12a of the stent 10, 10a in the stent delivery system 1, 1a, 1b, 1c described above, the configuration is not particularly limited to these. The anchor member 20, 20a may also be attached to the front portion of the stent 10, 10a. Further, although the anchor member 20, 20a is attached to the proximal end part 12, 12a of the stent 10, 10a in the stent delivery system 1, 1a, 1b, 1c described above, the configuration is not particularly limited to these. The anchor member 20, 20a may be attached to the distal portion or the middle portion of the stent 10, 10a.

As described above, in the case of the stent delivery system 1, 1a, 1b, 1c, the anchor member 20, 20a can be pushed in the direction of the front end (in the direction of the arrow 60) with the tapered portion 34b, 35b of the coil body 34, 35 since the anchor member 20, 20a of the stent 10, 10a is positioned between the tapered portion 34b, 35b of the coil body 34, 35 and the protruded portion 40, 40a when the stent 10, 10a is delivered to the target site with the pusher guide wire 30, 30a, 30b. Further, a rotating force exerted by an operator can be transmitted to the front ends of the pusher guide wire 30, 30a, 30b since the coil body 34, 35 extends to the front end joining portion 36 or the protruded portion 40a of the pusher guide wire 30, 30a, 30b. Furthermore, the stent 10, 10a in the middle of release can be retracted into the catheter 50 by pulling back the pusher guide wire 30, 30a, 30b in the direction of the proximal end (in the direction of the arrow 70) and allowing the protruded portion 40, 40a to make contact with the anchor member 20, 20a.

### DESCRIPTION OF SYMBOLS

- 1, 1a, 1b, 1c: Stent delivery system
- 10, 10a: Stent
- 12: Proximal end part of stent
- 20: Anchor member
- 30, 30a, 30b: Pusher guide wire
- 32, 32a: Core shaft
- 34,35: Coil body
- 34a, 35a: Small diameter portion
- 34b, 35b: Tapered portion
- 34c, 35c: Large diameter portion
- 36: Front end joining portion
- 38: Proximal end joining portion
- 40, 40a: Protruded portion
- 50: Catheter
- 52: Front end opening
- 54: Proximal end opening
- 60: Arrow (Direction toward front end)
- 70: Arrow (Direction toward proximal end)
- 80: Resin layer

## Claims

1. A stent delivery system (1, 1a, 1b, 1c) for delivering a stent (10, 10a) having an anchor member (20, 20a) to a target site with a pusher guide wire (30, 30a, 30b), the pusher guide wire (30, 30a, 30b) comprising:
a core shaft (32, 32a);
a coil body (34, 35) that covers a front end part of the core shaft (32, 32a) and that includes a tapered portion (34b, 35b) having an outer diameter decreasing toward a front end; and
a protruded portion (40, 40a) that fixes to an outer periphery of the coil body (34, 35) at a front end side relative to the tapered portion (34b, 35b) and that is capable of contacting on the anchor member (20, 20a), wherein
the anchor member (20, 20a) is positioned between the tapered portion (34b, 35b) and the protruded portion (40, 40a) when the stent (10, 10a) is delivered with the pusher guide wire (30, 30a, 30b).

2. The stent delivery system (1a) according to claim 1, wherein the anchor member (20a) is inclined in the same direction as the tapered portion (34b) of the coil body (34) relative to an axis (L) of the core shaft (32).

3. The stent delivery system (1b) according to claim 1 or claim 2, wherein a resin layer (80) is provided on an outer periphery of the tapered portion (34b).

4. The stent delivery system (1, 1a, 1b, 1e) according to any of claims 1 to 3, comprising a catheter (50) into which the pusher guide wire (30, 30a, 30b) holding the stent (10, 10a) can be inserted.

## Patentansprüche

1. Stentzuführsystem (1, 1a, 1b, 1c) zum Zuführen eines Stents (10, 10a) mit einem Ankerelement (20, 20a) zu einem Zielort mit einem Drückführungsdraht (30, 30a, 30b), wobei der Drückführungsdraht (30, 30a, 30b) Folgendes aufweist:
eine Kernwelle (32, 32a);
einen Wicklungskörper (34, 35), der einen vorderen Endteil der Kernwelle (32, 32a) bedeckt und der einen abgeschrägten Abschnitt (34b, 35b) mit einem Außendurchmesser hat, der zu einem vorderen Ende hin abnimmt; und
einen vorragenden Abschnitt (40, 40a), der an einem Außenumfang des Wicklungskörpers (34, 35) an einer vorderen Endseite relativ zu dem abgeschrägten Abschnitt (34b, 35b) fixiert ist und der zu einem Kontakt an dem Ankerelement (20, 20a) in der Lage ist, wobei
das Ankerelement (20, 20a) zwischen dem abgeschrägten Abschnitt (34b, 35b) und dem vorragenden Abschnitt (40, 40a) positioniert ist, wenn der Stent (10, 10a) mit dem Drückführungsdraht (30, 30a, 30b) zugeführt wird.

2. Stentzuführsystem (1a) gemäß Anspruch 1, wobei das Ankerelement (20a) in der gleichen Richtung wie der abgeschrägte Abschnitt (34b) des Wicklungskörpers (34) relativ zu einer Achse (L) der Kernwelle (32) geneigt ist.

3. Stentzuführsystem (1b) gemäß Anspruch 1 oder 2, wobei eine Kunststofflage (80) an einem Außenumfang des abgeschrägten Abschnittes (34b) vorgesehen ist.

4. Stentzuführsystem (1, 1a, 1b, 1c) gemäß einem der Ansprüche 1 bis 3, mit einem Katheter (50), in den der Drückführungsdraht (30, 30a, 30b), der den Stent (10, 10a) hält, eingeführt werden kann.

## Revendications

1. Système de pose de stent (1, 1a, 1b, 1c) pour poser un stent (10, 10a) ayant un élément d'ancrage (20, 20a) sur un site cible avec un fil-guide poussoir (30, 30a, 30b) ; le fil-guide poussoir (30, 30a, 30b) comprenant :
un arbre central (32, 32a) ;
un corps hélicoïdal (34, 35) qui recouvre une partie d'extrémité avant de l'arbre central (32, 32a) et qui comprend une partie progressivement rétrécie (34b, 35b) ayant un diamètre externe diminuant vers une extrémité avant ; et
une partie en saillie (40, 40a) qui se fixe sur une périphérie externe du corps hélicoïdal (34, 35) au niveau d'un côté d'extrémité avant par rapport à la partie progressivement rétrécie (34b, 35b) et qui est capable d'être en contact avec l'élément d'ancrage (20, 20a), dans lequel :
l'élément d'ancrage (20, 20a) est positionné entre la partie progressivement rétrécie (34b, 35b) et la partie en saillie (40, 40a) lorsque le stent (10, 10a) est posé avec le fil-guide poussoir (30, 30a, 30b).

2. Système de pose de stent (1a) selon la revendication 1, dans lequel l'élément d'ancrage (20a) est incliné dans la même direction que la partie progressivement rétrécie (34b) du corps hélicoïdal (34) par rapport à un axe (L) de l'arbre central (32).

3. Système de pose de stent (1b) selon la revendication 1 ou la revendication 2, dans lequel une couche de résine (80) est prévue sur une périphérie externe de la partie progressivement rétrécie (34b).

4. Système de pose de stent (1, 1a, 1b, 1c)) selon l'une quelconque des revendications 1 à 3, comprenant un cathéter (50) dans lequel le fil-guide poussoir (30, 30a, 30b) maintenant le stent (10, 10a) peut être inséré.
